# EUROPEAN PATENT SPECIFICATION

(11) **EP 1 165 069 B1**
(45) Date of publication and mention of the grant of the patent: **13.10.2004**
(21) Application number: 00903657.5
(22) Date of filing: 31.01.2000
(51) Int. Cl.: A61K 31/396, A61K 31/351, A61P 35/00, A61K 31/352

(54) **ANTITUMOUR SYNERGISTIC COMPOSITION**
SYNERGISTISCHE ZUSAMMENSETZUNGEN ZUR KREBSBEHANDLUNG
COMPOSITION SYNERGIQUE ANTI-TUMORALE

(30) Priority: 25.02.1999 GB 9904387
(43) Date of publication of application: 02.01.2002
(73) Proprietor: Pharmacia Italia S.p.A., 20152 Milano (IT)
(72) Inventor: GERONI, Cristina, I-20149 Milan (IT); RIPAMONTI, Marina, I-20162 Milan (IT); CARUSO, Michele, I-20131 Milan (IT); SUARATO, Antonino, I-20158 Milan (IT)
(74) Representative: Modiano, Micaela Nadia
(86) International application number: PCT/EP2000/000745
(87) International publication number: WO 2000/050032

(56) References cited:
- WO-A-99/48503
- US-A- 5 496 808
- US-A- 5 532 218
- EDER JP ET AL: "Sequence effect of irinotecan (CPT-11) and topoisomerase II inhibitors in vivo" CANCER CHEMOTHERAPY AND PHARMACOLOGY,DE,SPRINGER VERLAG, BERLIN, vol. 42, no. 4, 1998, pages 327-335, XP002112007 ISSN: 0344-5704

## Description

The present invention relates in general to the field of cancer treatment and, more particularly, provides an antitumor composition comprising an alkylating anthracycline and a topoisomerase II inhibitor, having a synergistic or additive antineoplastic effect.

The present invention provides, in a first aspect, a pharmaceutical composition for use in antineoplastic therapy in mammals, including humans, comprising
- an alkylating anthracycline of formula Ia or Ib : 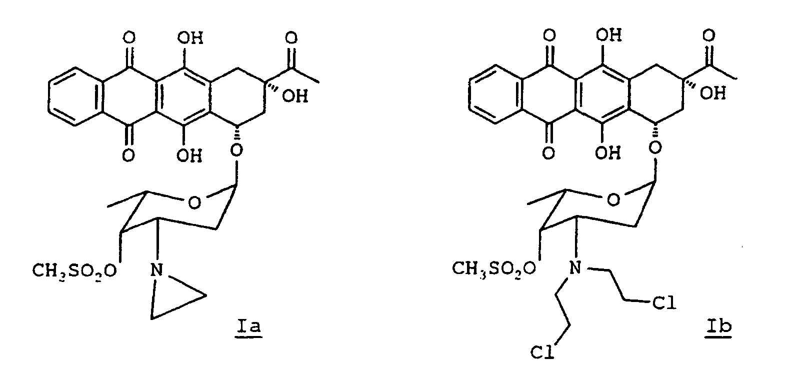
- an antineoplastic topoisomerase II inhibitor, and a pharmaceutically acceptable carrier or excipient.

The chemical names of the alkylating anthracyclines of formula Ia and Ib are 4-demethoxy-3'-deamino-3'-aziridinyl-4'-methansulfonyl daunorubicin (Ia) and 4-demethoxy-N,N-bis(2-chloroethyl)-4'-methansulfonyl daunorubicin (Ib). These alkylating anthracyclines were described in Anticancer Drug Design (1995), vol. 10, 641-653, and claimed respectively in US-A-5,532,218 and US-A-5,496,800. Both compounds intercalate into DNA via the chromophore and alkylate guanine at N⁷ position in DNA minor groove via their reactive moiety on position 3' of the amino sugar. Compounds Ia and Ib are able to circumvent the resistance to all major classes of cytotoxics, indicating that the compounds represent a new class of cytotoxic antitumor drugs.

Topoisomerase II inhibitors are described in various scientific publications. The main representatives of this wide class of drugs are: the anthracycline derivatives such as doxorubicin, daunorubicin, epirubicin, nemorubicin and idarubicin; the podophyllotoxin compounds etoposide and teniposide; the anthraquinone derivative like mitoxantrone and amsacrine. See for example the review: Cancer, Principles and Practice of Oncology, Lippincott-Raven Ed. (1997), 452-467. Doxorubicin and etoposide are the preferred topoisomerase II inhibitors to be used in the present invention. The present invention also provides a product comprising an alkylating anthracycline of formula Ia or Ib as defined above and an antineoplastic topoisomerase II inhibitor, as combined preparation for simultaneous, separate or sequential use in antitumor therapy.

A further aspect of the present invention relates to a method of treating a mammal including humans, suffering from a neoplastic disease state comprising administering to said mammal an alkylating anthracycline of formula Ia or Ib as defined above and an antineoplastic topoisomerase II inhibitor, in amounts effective to produce a synergistic antineoplastic effect.

The present invention also relates to a method for lowering the side effects caused by antineoplastic therapy with an antineoplastic agent in mammals, including humans, in need thereof, the method comprising administering to said mammal a combination preparation comprising an antineoplastic topoisomerase II inhibitor as defined above and an alkylating anthracycline of formula Ia or Ib, as defined above, in amounts effective to produce a synergistic antineoplastic effect.

By the term "a synergistic antineoplastic effect" as used herein is meant the inhibition of the growth tumor, preferably the complete regression of the tumor, administering an effective amount of the combination of an alkylating anthracycline of formula Ia or Ib as defined above and a topoisomerase II inhibitor to mammals, including human.

By the term "administered " or "administering" as used herein is meant parenteral and /or oral administration. By "parenteral" is meant intravenous, subcutaneus and intramuscolar administration. In the method of the subject invention, the alkylating anthracycline may be administered simultaneously with the compound with the topoisomerase II inhibitor activity, for example of the anthracycline or etoposide class, or the compounds may be administered sequentially, in either order. It will be appreciated that the actual preferred method and order of administration will vary according to, inter alia, the particular formulation of the alkylating anthracycline of formula Ia or Ib being utilized, the particular formulation of the topoisomerase II inhibitor, such as one of the anthracycline or etoposide class, being utilized, the particular tumor model being treated, and the particular host being treated.

For the administration of the alkylating anthracycline of formula Ia or Ib, the course of therapy generally employed is from 0.1 to 200 mg/m² of body surface area. More preferably, the course therapy employed is from 1 to 50 mg/m² of body surface area.

For the administration of the topoisomerase II inhibitor the course of therapy generally employed is from 1 to 1000 mg/m² of body surface area. More preferably, the course therapy employed is from 10 to 500 mg/m² of body surface area. The antineoplastic therapy is in particular suitable for treating breast, ovary lung, colon, kidney, stomach, pancreas, liver, melanoma, leukemia and brain tumors in mammals, including humans.

In a further aspect, the present invention is directed to the preparation of a pharmaceutical composition containing an effective amount of an alkylating anthracycline of formula Ia or Ib as defined above and an antineoplastic topoisomerase II inhibitor in the prevention or treatment of metastasis or for the treatment of tumors by angiogenesis inhibition, as well as to the use of an alkylating anthracycline of formula Ia or Ib as defined above and an antineoplastic topoisomerase II inhibitor for the treatment of tumors by angiogenesis inhibition or for the treatment or prevention of metastasis .

As stated above, the effect of an alkylating anthracycline of formula Ia or Ib and a topoisomerase II inhibitor, such as an anthracycline or etoposide derivative, is significantly increased without a parallel increased toxicity. In other words, the combined therapy of the present invention enhances the antitumoral effects of the alkylating anthracycline and of the topoisomerase II inhibitor and thus yields the most effective and least toxic treatment for tumors. The superadditive actions of the combination preparation of the present invention are shown for instance by the following *in vivo* tests, which are intended to illustrate the present invention.

Table 1 shows the antileukemic activity on disseminated L1210 murine leukemia obtained combining Ia with etoposide.

At the dose of 30 mg/kg of etoposide alone (day +3) and at the dose of 1 mg/kg of Ia alone (days +1,2) were associated, without toxicity, with ILS% values of 100 and 67, respectively. Combining etoposide and Ia at the same doses with the same schedule an increase of activity with ILS% values of 450 was observed, indicating a synergistic effect.

Table 2 shows the antileukemic activity on disseminated L1210 murine leukemia obtained combining Ia with doxorubicin. At the dose of 13 mg/kg of doxorubicin alone (day +3) and at the dose of 1.5 mg/kg of Ia alone (days +1,2) were associated, without toxicity, with ILS% values of 50 and 67, respectively. Combining doxorubicin and Ia at the same doses with the same schedule an increase of activity with ILS% values of 150 was observed, indicating a synergistic effect.

For these experiments Ia was solubilized in [Cremophor® /EtOH= 6.5:3.5]/[normal saline]=20/80 v/v, while standard etoposide pharmaceutical preparation and doxorubicin solubilized in water were used.

**Table 1:**

| Antileukemic activity against disseminated L1210¹ murine leukemia of Ia in combination with Etoposide | | | | | |
|---|---|---|---|---|---|
| Compound | Treatment schedule | Dose² (mg/kg/day) | ILS%³ | Tox ⁴ | LTS⁵ |
| Ia | iv +1,2 | 1 | 67 | 0/10 | 0/10 |
| Etoposide | iv +3 | 30 | 100 | 0/10 | 0/10 |
| Ia + Etoposide | iv +1,2 iv +3 | 1 + 30 | 450 | 0/10 | 4/10 |

**Table 2:**

| Antileukemic activity against disseminated L1210¹ murine leukemia of Ia in combination with Doxorubicin | | | | | |
|---|---|---|---|---|---|
| Compound | Treatment schedule | Dose² (mg/kg/day) | ILS%³ | Tox ⁴ | LTS⁵ |
| Ia | iv +1,2 | 1.5 | 67 | 0/10 | 0/10 |
| Doxorubicin | iv +3 | 13 | 50 | 0/10 | 0/10 |
| Ia + Doxorubicin | iv +1,2 iv +3 | 1.5 + 13 | 150 | 0/10 | 3/10 |

| | | | | | |
|---|---|---|---|---|---|
| 1)L1210 leukemia cells (10⁵/mouse) are injected iv on day 0. | | | | | |
| 2) Treatment is given starting on day 1 after tumor transplantation (day 0). | | | | | |
| 3)Increase in life span: [(median survival time of treated mice/median survival time of controls)x 100]-100 | | | | | |
| 4)Number of toxic deaths/number of mice. | | | | | |
| 5) Long Term Survivors (>60 days) at the end the experiment. | | | | | |

## Claims

1. Products containing an alkylating anthracycline of formula Ia or Ib: 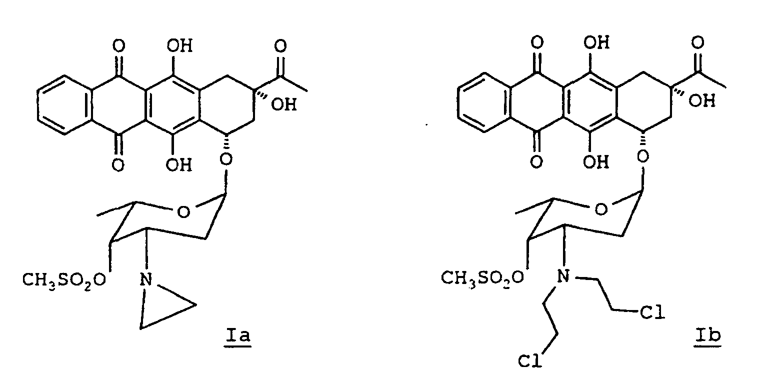 and an antineoplastic topoisomerase II inhibitor as a combined preparation for simultaneous, separate or sequential use in the treatment of tumors.

2. Products according to claim 1 wherein the alkylating anthracycline is 4-demethoxy-3'-deamino-3'-aziridinyl-4'-methansulfonyl daunorubicin.

3. Products according to claim 1 or 2 wherein the topoisomerase II inhibitor is etoposide.

4. Products according to claim 1 or 2 wherein the topoisomerase II inhibitor is doxorubicin.

5. A pharmaceutical composition comprising a pharmaceutically acceptable carrier or excipient and, as active ingredient, an alkylating anthracycline of formula Ia or Ib as defined in claim 1 and an antineoplastic topoisomerase II inhibitor.

6. A composition according to claim 5 wherein the topoisomerase II inhibitor is doxorubicin or etoposide.

7. Use of an alkylating anthracycline of formula Ia or Ib as defined in claim 1 and an antineoplastic topoisomerase II inhibitor in the preparation of a medicament for use in the treatment of tumors.

8. Use according to claim 7 wherein the topoisomerase II inhibitor is etoposide or doxorubicin.

9. Use of an alkylating anthracycline of formula Ia or Ib as defined in claim 1 and an antineoplastic topoisomerase II inhibitor in the preparation of a medicament for use in the prevention or treatment of metastasis or in the treatment of tumors by inhibition of angiogenesis.

## Patentansprüche

1. Produkte, die ein alkylierendes Anthracyclin der Formel Ia oder Ib: 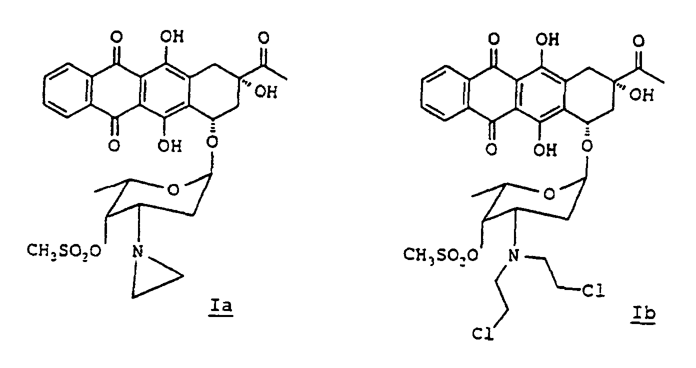 und einen antineoplastischen Topoisomerase II Hemmer als eine kombinierte Zubereitung für die gleichzeitige, getrennte oder aufeinander folgende Verwendung in der Behandlung von Tumoren enthalten.

2. Produkte gemäß Anspruch 1, worin das alkylierende Anthracyclin 4-Desmethoxy-3'-desamino-3'-aziridinyl-4'methansulfonyl Daunorubicin ist.

3. Produkte gemäß Anspruch 1 oder 2, worin der Topoisomerase II Hemmer Etoposid ist.

4. Produkte gemäß Anspruch 1 oder 2, worin der Topoisomerase II Hemmer Doxorubicin ist.

5. Eine pharmazeutische Zusammensetzung, die einen pharmazeutisch verträglichen Träger oder ein Bindemittel und, als aktiven Inhaltsstoff, ein alkylierendes Anthracyclin gemäß Formel Ia oder Ib, wie in Anspruch 1 definiert, und einen antineoplastischen Topoisomerase II Hemmer umfasst.

6. Eine Zusammensetzung gemäß Anspruch 5, worin der Topoisomerase II Hemmer Doxorubicin oder Etoposid ist.

7. Verwendung eines alkylierenden Anthracyclins gemäß Formel Ia oder Ib, wie in Anspruch 1 definiert, und eines antineoplastischen Topoisomerase II Hemmers in der Herstellung eines Medikaments für die Verwendung in der Behandlung von Tumoren.

8. Verwendung gemäß Anspruch 7, worin der Topoisomerase II Hemmer Etoposid oder Doxorubicin ist.

9. Verwendung eines alkylierenden Anthracyclins gemäß Formel Ia oder Ib, wie in Anspruch 1 definiert, und eines antineoplastischen Topoisomerase II Hemmers in der Herstellung eines Medikaments zur Verwendung in der Prävention oder der Behandlung von Metastasen oder in der Behandlung von Tumoren durch Hemmung der Angiogenese.

## Revendications

1. Produits contenant une anthracycline alkylante de formule Ia ou Ib : 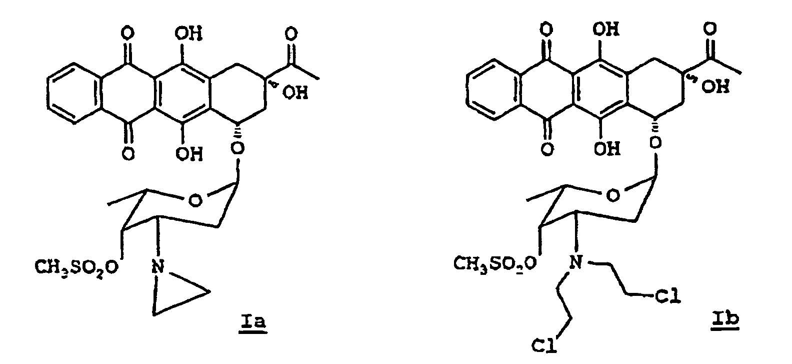 et un inhibiteur antinéoplasique de topoisomérase II comme préparation combinée destinée à l'administration simultanée, séparée ou séquentielle dans le traitement de tumeurs.

2. Produits suivant la revendication 1, dans lesquels l'anthracycline alkylante est la 4-déméthoxy-3'-déamino-3'-aziridinyl-4'-méthanesulfonyl-daunorubicine.

3. Produits suivant la revendication 1 ou 2, dans lesquels l'inhibiteur de topoisomérase II est l'étoposide.

4. Produits suivant la revendication 1 ou 2, dans lesquels l'inhibiteur de topoisomérase II est la doxorubicine.

5. Composition pharmaceutique comprenant un support ou excipient acceptable du point de vue pharmaceutique et, comme ingrédient actif, une anthracycline alkylante de formule Ia ou Ib telle que définie dans la revendication 1 et un inhibiteur antinéoplasique de topoisomérase II.

6. Composition suivant la revendication 5, dans laquelle l'inhibiteur de topoisomérase II est la doxorubicine ou l'étoposide.

7. Utilisation d'une anthracycline alkylante de formule Ia ou Ib telle que définie dans la revendication 1 et d'un inhibiteur antinéoplasique de topoisomérase II dans la préparation d'un médicament destiné à être utilisé dans le traitement de tumeurs.

8. Utilisation suivant la revendication 7, dans laquelle l'inhibiteur de topoisomérase II est l'étoposide ou la doxorubicine.

9. Utilisation d'une anthracycline alkylante de formule Ia ou Ib telle que définie dans la revendication 1 et d'un inhibiteur antinéoplasique de topoisoimérase II dans la préparation d'un médicament destiné à être utilisé dans la prévention ou le traitement de métastases ou dans le traitement de tumeurs par inhibition de l'angiogenèse.
